# EUROPÄISCHE PATENTSCHRIFT

(11) **EP 0 367 907 B1**
(45) Veröffentlichungstag und Bekanntmachung des Hinweises auf die Patenterteilung: **27.10.1993**
(21) Anmeldenummer: 89113127.8
(22) Anmeldetag: 18.07.1989
(51) Int. Cl.: G01N 15/05, B01L 3/02, A61B 5/14

(54) **Vorrichtung zum Messen der Blutkörperchensenkungsgeschwindigkeit**
Device for measuring the sedimentation speed of blood particles
Dispositif pour la mesure de la vitesse de sédimentation de corpuscules sanguins

(30) Priorität: 11.11.1988 DE 3838313
(43) Veröffentlichungstag der Anmeldung: 16.05.1990
(73) Patentinhaber: KABE Labortechnik Gesellschaft mit beschränkter Haftung, D-51588 Nümbrecht (DE)
(72) Erfinder: Bethkenhagen,Jürgen, W-5223 Nümbrecht (DE); Kolpe,Dieter, W-5276 Wiehl 1 (DE)
(74) Vertreter: Stenger, Watzke & Ring Patentanwälte

(56) Entgegenhaltungen:
- DE-A- 2 147 824
- DE-A- 3 025 800
- DE-A- 3 505 783

## Beschreibung

Die Erfindung betrifft eine Vorrichtung zum Messen der Blutkörperchensenkungsgeschwindigkeit (BSG) einer ungerinnbar gemachten Blutprobe mittels eines Senkungsrohres unter Verwendung einer Entnahmespritze, wobei das Senkungsrohr zugleich auch die den Spritzenkolben tragende Spritzenkolbenstange der Entnahmespritze bildet und wobei zum Umfüllen der aufgezogenen Blutprobe diese beim Verschieben des Spritzenkolbens in die umgekehrte Aufziehrichtung von der Probenkammer der Entnahmespritze in das Senkungsrohr gedrückt wird.

Nach dem Handbuch E. Merck "Klinisches Labor", 12. Auflage, 1974, Seiten 49 bis 51, wird bei der BSG die Sedimentationsgeschwindigkeit der Erythrozyten des ungerinnbar gemachten Blutes in einer senkrecht aufgehängten, von 0 bis 200 graduierten Sedimentierungspipette mit gleichbleibender Kalibrierung gemessen. Dazu wird die Blutprobe auf eine mit einem Gerinnungshemmer präparierten Entnahmespritze aufgezogen und anschließend in die Sedimentierungspipette umgefüllt. Nach 1 und 2 Stunden werden die Senkungswerte in der Grenze zwischen Erythrozytensäule und Plasmaschicht abgelesen. Die BSG erlaubt in der Diagnostik wichtige Nebenbeobachtungen.

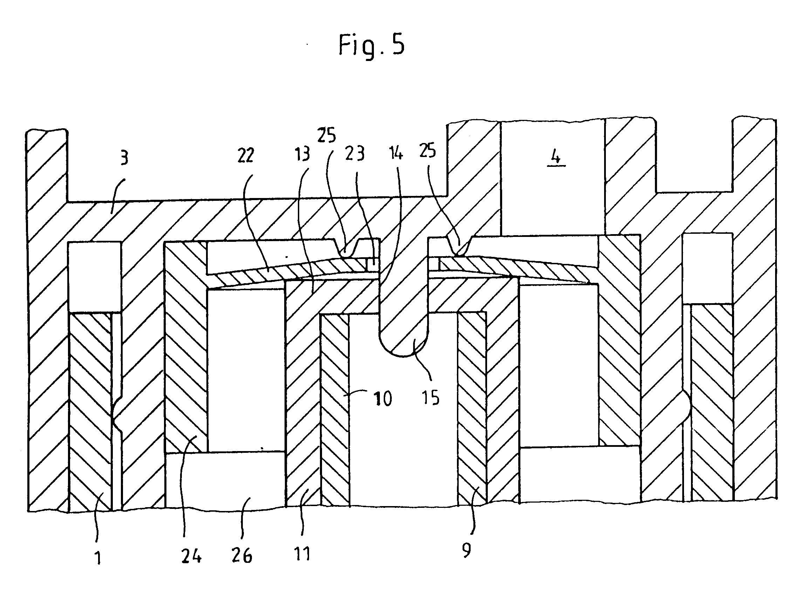

Aus der DE-OS 30 25 800 ist eine Vorrichtung zum Messen der Blutkörperchensenkungsgeschwindigkeit (BSG) einer mittels eines Blutgerinnungshemmers ungerinnbar gemachten Blutprobe unter Verwendung eines geschlossenen Systems der eingangs genannten Art bekannt (Fig. 5a bis 5c). Zu diesem Zweck ist eine (eine Aufziehöffnung mit einer Kanüle aufweisende) Entnahmespritze vorgesehen, mittels der die Blutprobe in eine Probenkammer aufziehbar ist. Dafür ist innerhalb der Entnahmespritze ein Spritzenkolben vorgesehen, welcher mittels einer aus dem hinteren Ende der Entnahmespritze herausragenden Spritzenkolbenstange verschiebbar ist. Die Spritzenkolbenstange bildet dabei gleichzeitig das durchsichtige Senkungsrohr für den Messvorgang. Mit seinem unteren, offenen Ende mündet dabei das Senkungsrohr in der Probenkammer der Entnahmespritze, so daß ein Umfüllen der aufgezogenen Blutprobe in das Senkungsrohr möglich ist, wenn das Senkungsrohr in die entgegengesetzte Aufziehrichtung verschoben wird und dabei der Spritzenkolben die Blutprobe aus der Probenkammer in das Senkungsrohr drückt.

Bei dieser bekannten Vorrichtung zum Messen der Blutkörperchensenkungsgeschwindigkeit (BSG) ist zunächst von Nachteil, daß das Senkungsrohr bei der Handhabung der Entnahmespritze insbesondere durch das weit auskragende Ende zerbrochen werden kann. Vor allem aber ist bei der bekannten Messvorrichtung von Nachteil, daß das in die Probenkammer der Entnahmespritze ragende offene Ende des Senkungsrohres immer offen ist, so daß insbesondere bei der noch unbenutzten Messvorrichtung bzw. Entnahmespritze der Blutgerinnungshemmer in das Senkungsrohr fließen kann. Darüber hinaus ist bei Durchführung der Messung das untere Ende des Senkungsrohres offen, so daß nachteiligerweise die Messprobe unten austreten und somit das Messergebnis verfälschen kann.

Aus der DE-PS 29 35 371 ist eine Messvorrichtung für die Blutkörperchensenkungsgeschwindigkeit (BSG) bekannt, bei der die Blutprobe durch ein Reagenzglas aufgenommen ist. Um diese Blutprobe in ein Senkungsrohr umfüllen zu können, ist eine auf das Senkungsrohr aufgeschobene Hülse vorgesehen, die hinwiederum in das Reagenzglas derart eingeschoben wird, daß diese als Kolben wirkend die Blutprobe in das Senkungsrohr drückt. Der Nachteil dieser Messvorrichtung liegt darin, daß es sich um ein offenes System handelt, bei dem Blut austreten und somit die Bedienungspersonen mit Krankheitserregern infizieren kann.

Ausgehend von einer Messvorrichtung der eingangs angegebenen Art liegt der Erfindung die **Aufgabe** zugrunde, diese bekannte Messvorrichtung derart weiterzubilden, daß vor allem in der Grundstellung der Entnahmespritze bei eingeschobenem Spritzenkolben das in die Probenkammer mündende offene Ende des Senkungsrohres geschlossen ist.

Als technische **Lösung** wird mit der Erfindung vorgeschlagen, daß das Senkungsrohr im Nichtablesebereich eines Gestells von einer die eigentliche Spritzenkolbenstange mit dem Spritzenkolben bildenden Hülse umgeben ist, daß die Hülse an ihrem unteren Ende durch einen eine mittige Öffnung aufweisenden, angeformten Ansatz abgeschlossen ist, bis zu dem das Senkungsrohr in die Hülse hineinsteckbar ist, und daß der Öffnung ein diese im hineingeschobenen Zustand des Spritzenkolbens verschließendes Verschlußelement zugeordnet ist.

Eine nach dieser technischen Lehre ausgebildete Messvorrichtung zum Bestimmen der Blutkörperchensenkungsgeschwindigkeit (BSG) hat zunächst den Vorteil, daß durch die vorzugsweise aus Kunststoff bestehende Hülse das Senkungsrohr verstärkt wird und nicht so leicht insbesondere im Austrittsbereich aus der Entnahmespritze zerbrechen kann. Vor allem aber hat die erfindungsgemäße Messvorrichtung den Vorteil, daß durch das Verschlußelement in der Grundstellung der Entnahmespritze mit eingeschobenem Spritzenkolben die in die Probenkammer mündende Öffnung des Senkungsrohres verschlossen wird, so daß einerseits vor dem Gebrauch der Messvorrichtung bzw. der Entnahmespritze der Blutgerinnungshemmer nicht in das Senkungsrohr eintreten kann und andererseits während des Messvorganges die Messprobe nicht aus dem unteren Ende des Senkungsrohres austreten kann.

In einer ersten Ausführungsform ist der Ansatz durch eine Ringscheibe gebildet, das Senkungsrohr ist dauerhaft von der Hülse aufgenommen und im Boden der Entnahmespritze ist ein ins Innere der Probenkammer ragender, das Verschlußelement bildender Verschlußstift angeordnet, der in der unteren Position des Senkungsrohres die Öffnung dichtend verschließt. In dieser Ausführungsform bildet somit die Hülse zusammen mit dem Senkungsrohr eine bauliche Einheit, bei der das Senkungsrohr bis zur den Anschlag definierenden Ringscheibe in die Hülse hineingesteckt ist. Um das Senkungsrohr sicher in der Hülse zu halten, ist letztere im Bereich der Ringscheibe vorzugsweise etwas konisch verjüngt. Der Verschluß wird dabei durch den Verschlußstift zusammen mit der Öffnung in der Ringscheibe gebildet, so daß auf technisch einfache Weise das untere offene Ende des Senkungsrohres in der Grundposition der Entnahmespritze verschlossen werden kann.

Da bei der ersten Ausführungsform der Messvorrichtung beim Aufziehen der Blutprobe diese bereits teilweise in das Senkungsrohr fließen kann und somit mit dem Blutgerinnungshemmer nicht in Berührung gelangt, wird in einer zweiten, bevorzugten Ausführungsform der erfindungsgemäßen Messvorrichtung vorgeschlagen, daß der Ansatz durch einen Hohlzylinder gebildet ist, daß das Senkungsrohr bis zum Hohlzylinder in die Hülse hineinsteckbar und wieder herausziehbar ist und daß in der Öffnung des Hohlzylinders ein durch Hineinstecken des Senkungsrohres in die Hülse sowie durch Anlage am Boden der Entnahmespritze entsprechend verschiebbarer, das Verschlußelement bildender Verschlußstopfen angeordnet ist, der in der durch das Senkungsrohr hinausgedrückten Verschiebestellung einen Durchströmkanal zwischen dem Innern des Senkungsrohres und der Probenkammer öffnet und ansonsten verschließt. Bei dieser Ausführungsform bildet die Hülse sowie das Senkungsrohr keine bauliche Einheit, sondern das Senkungsrohr kann aus der Hülse ohne weiteres herausgezogen werden. Dies bedeutet, daß in der Grundposition der Entnahmespritze, bei der die vordere Öffnung der Hülse durch den Verschlußstopfen verschlossen ist, das Senkungsrohr vollständig aus der Hülse herausgezogen ist. In diesem Zustand kann durch Nachhintenziehen der Hülse die Blutprobe aufgezogen werden, wobei durch den Verschlußstopfen die Blutprobe nicht in die Hülse dringen kann. Nach Aufziehen der Blutprobe wird dann das Senkungsrohr in die Hülse eingeführt und bis zum vorderen Ende derart verschoben, daß der Verschlußstopfen nach vorne gedrückt wird und den Durchströmkanal zwischen dem Innern des Senkungsrohres und der Probenkammer der Entnahmespritze öffnet. In diesem Zustand kommt das vordere Ende des Senkungsrohres am Hohlzylinder zur Anlage, der somit gewissermaßen als Anschlag wirkt. Nach Verschließen der Aufziehöffnung der Entnahmespritze kann dann nach Vermischen der Blutprobe mit dem Blutgerinnungshemmer diese umgefüllt werden, indem die Hülse in die umgekehrte Aufziehrichtung gedrückt wird, so daß die Blutprobe nunmehr durch den geöffneten Durchströmkanal in das Senkungsrohr fließen kann. Durch weiteres Verschieben des Senkungsrohres wird dann der Verschlußstopfen wieder hineingedrückt und verschließt die Öffnung im Hohlzylinder. Anschließend kann dann die Messung durchgeführt werden.

Vorzugsweise ist der Durchströmkanal im Verschlußstopfen ausgebildet, indem dieser auf seiner Mantelfläche vorzugsweise koaxiale Nuten aufweist.

Um den Verschlußstopfen nach dem Umfüllen der Blutprobe wieder sicher in die Verschlußstellung verschieben zu können, ist vorzugsweise im Boden der Entnahmespritze ein Anschlagstift für den Verschlußstopfen angeordnet.

In einer Weiterbildung der Hülse weist diese im Innern der Entnahmespritze einen Anschlag auf. Dadurch ist der Hubbereich des Spritzenkolbens vorbestimmt. Dieser Anschlag kann beispielsweise durch eine Verstärkung der Wanddicke der Hülse erzielt werden, wobei der Durchmessersprung den Anschlag bildet.

Eine weitere Weiterbildung schlägt vor, daß die Hülse außerhalb der Entnahmespritze eine Handhabe aufweist, damit der Benutzer das Senkungsrohr besser verschieben kann.

In einer bevorzugten Ausführungsform ist der Spritzenkolben durch eine Erweiterung der Hülse gebildet, so daß auf herstellungstechnisch einfache Weise die Spritzenkolbenstange/Spritzenkolben-Einheit geschaffen ist.

Dabei weist vorzugsweise die Erweiterung der Hülse eine zum Probenraum hin offene zylinderringförmige Tasche zur Aufnahme eines Blutgerinnungshemmers auf. Dadurch erhält der Kolben eine Dreifachfunktion, indem er zusätzlich zum Aufziehen und Umfüllen der Blutprobe noch als Aufbewahrungsbehälter für den Blutgerinnungshemmer zum Ungerinnbarmachen der Blutprobe dient.

In einer bevorzugten Weiterbildung kann zum Verschließen der Aufziehöffnung der Entnahmespritze zusätzlich noch ein Rückschlagventil vorgesehen sein, das in einer praktischen Ausführungsform aus einer elastisch verformbaren Membrane mit einer Ventilöffnung sowie einem im Boden der Entnahmespritze angeformten, dazu korrespondierenden Ventilsitz besteht. Dieses Rückschlagventil öffnet beim Aufziehen der Blutprobe in die Entnahmespritze, während es beim Umfüllen der Blutprobe in das Senkungsrohr verhindert, daß die Blutprobe durch die Aufziehöffnung der Entnahmespritze hindurchgedrückt wird.

Zwei Ausführungsbeispiele einer erfindungsgemäßen Vorrichtung zum Messen der BSG werden nachfolgend anhand der Zeichnungen beschrieben. In diesen zeigt:
- Fig. 1: einen Schnitt durch ein erstes Ausführungsbeispiel einer Vorrichtung zum Messen der BSG im Ursprungszustand vor dem Aufziehen einer Blutprobe, anhand der die BSG gemessen werden soll;
- Fig. 2: eine teilweise geschnittene Ansicht der Vorrichtung in Fig. 1 nach Aufziehen einer Blutprobe;
- Fig. 3: die Vorrichtung in Fig. 2, nachdem die Blutprobe in das Senkungsrohr umgefüllt und die Vorrichtung an einem Gestell mit graduierter Skala zum Messen der BSG aufgehängt worden ist;
- Fig. 4a: die Vorrichtung in Fig. 1 in vergrößertem Maßstab im Bereich der Entnahmespritze;
- Fig. 4b: eine ebenfalls vergrößerte Darstellung des unteren Endes des Senkungsrohres in Fig. 1;
- Fig. 5: eine nochmals vergrößerte Darstellung der Vorrichtung in Fig. 1 im Bereich des Spritzenkopfes;
- Fig. 6: einen Schnitt durch ein zweites Ausführungsbeispiel einer Messvorrichtung entsprechend der Darstellung in Fig. 4a;
- Fig. 7: eine vergrößerte Darstellung der Vorrichtung in Fig. 6 im Bereich des Spritzenkopfes;
- Fig. 8: eine teilweise geschnittene Darstellung während des Umfüllens der Blutprobe aus der Probenkammer der Entnahmespritze in das Senkungsrohr;
- Fig. 9a: eine Ansicht einer ersten Ausführungsform eines Verschlußstopfens;
- Fig. 9b: einen Schnitt durch den Verschlußstopfen in Fig. 9a;
- Fig. 9c: eine Ansicht von oben auf den Verschlußstopfen in Fig. 9a;
- Fig. 10a: eine Ansicht einer zweiten Ausführungsform eines Verschlußstopfens;
- Fig. 10b: einen Schnitt durch den Verschlußstopfen in Fig. 10a;
- Fig. 10C: eine Ansicht von oben auf den Verschlußstopfen in Fig. 10a;
- Fig. 11a: eine Ansicht einer dritten Ausführungsform eines Verschlußstopfens;
- Fig. 11b: einen Schnitt durch den Verschlußstopfen in Fig. 11a;
- Fig. 11c: eine Ansicht von oben auf den Verschlußstopfen in Fig. 11a.

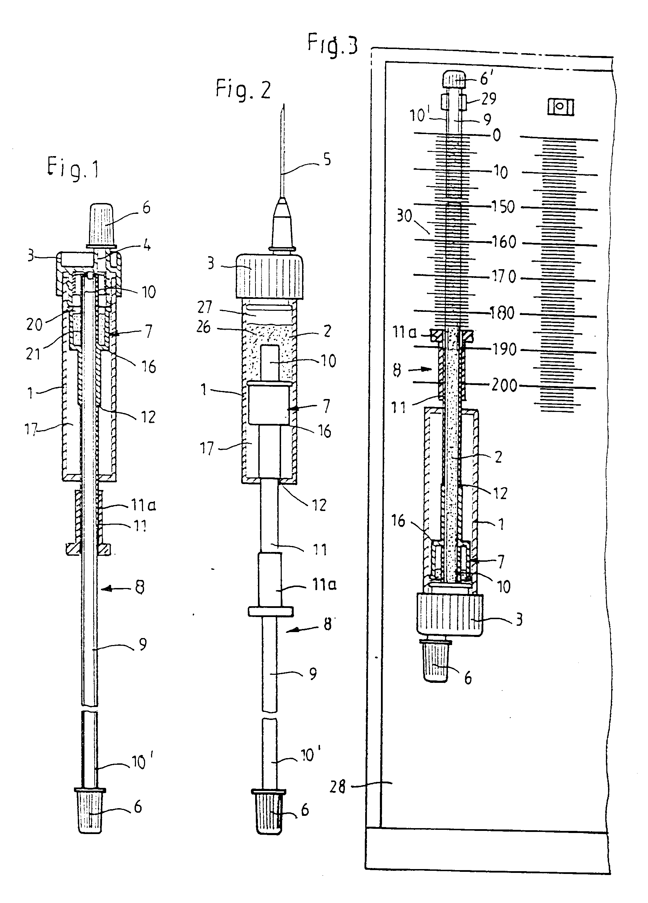

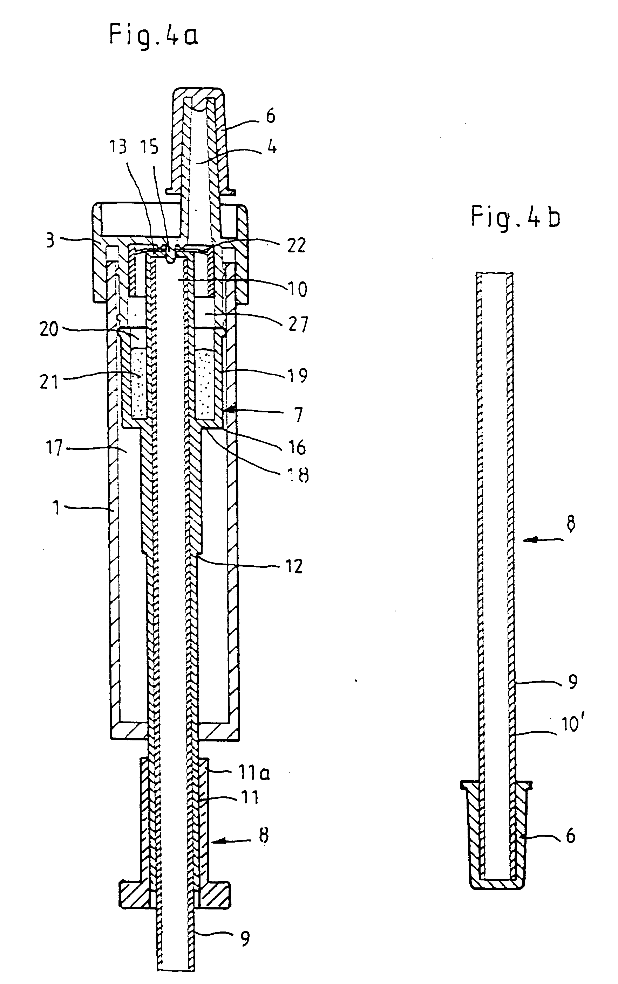

Eine Vorrichtung zum Messen des BSG besteht aus einer Entnahmespritze 1, mittels der einem Patienten eine Blutprobe 2 entnommen werden kann. Zu diesem Zweck ist auf das offene Ende der Entnahmespritze 1 ein Anschlußstück 3 dichtend aufgesetzt. Dieses weist eine konisch sich verjüngende Aufziehöffnung 4 auf, auf die eine Kanüle 5 aufgesteckt werden kann, wie in Fig. 2 zu erkennen ist. Im Ursprungszustand der Entnahmespritze 1 ist jedoch diese Aufziehöffnung 4 durch ein Verschlußkäppchen 6 verschlossen, wie Fig. 1 sowie Fig. 4a zeigt.

Im Innern der Entnahmespritze 1 ist ein Spritzenkolben 7 mittels einer Spritzenkolbenstange 8 verschiebbar geführt, wobei die Spritzenkolbenstange 8 in der üblichen Weise durch das hintere Ende der Entnahmespritze 1 hindurchgeführt ist.

Die Spritzenkolbenstange 8 ist durch ein Senkungsrohr 9 aus durchsichtigem Material, beispielsweise Kunststoff oder Glas, gebildet. Sowohl das untere Ende 10 als auch das obere Ende 10′ des Senkungsrohres 9 ist dabei jeweils offen und kann bei Bedarf verschlossen werden, wie nachfolgend noch näher ausgeführt werden wird. Unter "unteres Ende 10" des Senkungsrohres 9 ist dabei dasjenige Ende zu verstehen, welches in das Innere der Entnahmespritze 1 ragt, welches also beim Durchführen der Messung der BSG unten ist. Entsprechend ist das "obere Ende 10′" des Senkungsrohres 9 das hintere Ende des Senkungsrohres 9, das aus der Entnahmespritze 1 herausragt und das bei der Messung der BSG oben liegt.

Das Senkungsrohr 9 ist in demjenigen Bereich, in dem nicht abgelesen wird, von einer Hülse 11 umgeben, die das Senkungsrohr 9 verstärkt. Der Hülsenbereich außerhalb der Entnahmespritze 1 dient dabei als Griffbereich, wobei eine auf dem Hülsenende befestigte Handhabe 11a als Betätigungsgriff für den Benutzer dient. Im Innern der Entnahmespritze 1 ist durch eine Verdickung der Wandstärke der Hülse 11 ein Anschlag 12 gebildet, der das Nachhintenziehen des Spritzenkolbens 7 begrenzt. Im Bereich des unteren Endes 10 des Senkungsrohres 9 weist die Hülse 11 eine einstückig angeformte Ringscheibe 13 mit einer mittigen runden Öffnung 14 auf. In die so ausgebildete Hülse 11 ist das Senkungsrohr 9 bis zur Ringscheibe 13 hin vollständig dauerhaft hineingesteckt. Korrespondierend zur Öffnung 14 in der Ringscheibe 13 weist der Boden des Anschlußstücks 3 einen angeformten Verschlußstift 15 auf, der in der vorderen Stellung der Hülse 11 in dessen Öffnung 14 ragt und diese dichtend abschließt.

Die Hülse 11 ist weiterhin mit einer Erweiterung 16 versehen, die den Spritzenkolben 7 bildet. Dieser dichtet den Ringraum 17 zwischen der Spritzenkolbenstange 8 (Senkungsrohr 9 sowie Hülse 11) und der Innenwand der Entnahmespritze 1 ab. Die Erweiterung 16 der Hülse 11 ist dabei durch eine einstückig angeformte, in radialer Richtung sich erstreckende Ringscheibe 18 gebildet, an der hinwiederum ein Zylindermantel 19 angeformt ist. Dieser definiert zwischen sich und der Hülse 11 eine zylinderringförmige Tasche 20, die der Aufnahme eines Blutgerinnungshemmers 21 dient.

Schließlich ist im vorderen Bereich der Entnahmespritze 1 noch ein Rückschlagventil angeordnet. Dieses besteht aus einer elastisch verformbaren Membrane 22 mit einer mittigen Ventilöffnung 23, deren Durchmesser etwas größer ist als der Durchmesser des Verschlußstiftes 15. Diese Membrane 22 ist durch einen zylindermantelförmigen Haltering 24 im Anschlußstück 3 der Entnahmespritze 1 gehalten. Korrespondierend zu der Ventilöffnung 23 der Membrane 22 ist im Boden des Anschlußstückes 3 ein ringförmiger Ventilsitz 25 angeformt, welcher die Ventilöffnung 23 umgibt.

Diese Arbeits- und Funktionsweise der Vorrichtung zum Messen der BSG wird nachfolgend beschrieben:

In Fig. 1 ist die Entnahmespritze 1 im Ursprungszustand dargestellt, also mit nach vorne geschobenem Spritzenkolben 7. Sowohl die Aufziehöffnung 4 des Anschlußstücks 3 als auch das hintere (obere) Ende 10′ des Senkungsrohres 9 ist jeweils mit einem Verschlußkäppchen 6 verschlossen.

Zur Entnahme einer Blutprobe 2 wird das Verschlußkäppchen 6 von der Aufziehöffnung 4 des Anschlußstücks 3 abgenommen und statt dessen die Kanüle 5 aufgesteckt. Das Verschlußkäppchen 6 auf dem hinteren Ende des Senkungsrohres 9 wird dabei belassen. Durch Zurückziehen der Spritzenkolbenstange 8 bis zum Anschlag 12 der Hülse 11 wird die Blutprobe 2 aufgezogen. Dabei hebt die Membrane 22 des Rückschlagventils vom Ventilsitz 25 ab, so daß das Blut durch das Anschlußstück 3 hindurch sowie durch die Ventilöffnung 23 der Membrane 22 in die Probenkammer 26 fließen kann. Da sich in dieser ein Luftpolster 27 befindet, kann die Blutprobe 2 durch Schütteln mit dem Blutgerinnungshemmer 21 vermischt werden. Dies ist in Fig. 2 dargestellt.

Anschließend wird die Kanüle 5 wieder abgenommen und die Aufziehöffnung 4 des Anschlußstücks 3 durch das Verschlußkäppchen 6 verschlossen. Die Entnahmespritze 1 wird anschließend umgedreht, so daß das hintere Ende des Senkungsrohres 9 oben ist. Das das obere Ende 10′ des Senkungsrohres 9 verschließende Verschlußkäppchen 6 wird abgenommen, so daß die Blutprobe 2 aus der Probenkammer 26 in das Senkungsrohr 9 umgefüllt werden kann. Zu diesem Zweck wird die Spritzenkolbenstange 8 nach unten verschoben, so daß der Spritzenkolben 7 die Blutprobe 2 aus der Probenkammer 26 in das Senkungsrohr 9 drückt, wobei der Blutspiegel innerhalb des Senkungsrohres 9 allmählich bis zu einer vorgegebenen Nullmarke ansteigt. Bei diesem Nachun - tendrücken der Spritzenkolbenstange 8 schließt das Rückschlag ventil, indem die Membrane 22 am Ventilsitz 25 zur Anlage kommt, so daß das Blut nicht aus der Aufziehöffnung 4 des Anschlußstücks 3 austreten kann. Da das Volumen der Probenkammer 26 auf das Volumen innerhalb des Senkungsrohres 9 abgestimmt ist, befindet sich der Spritzenkolben 7 bei gefülltem Senkungsrohr 9 in der untersten Stellung, in der der Verschlußstift 15 die Öffnung 14 der Hülse 11 zusätzlich verschließt.

Nachdem das Senkungrohr 9 mit der Blutprobe 2 bis zur Nullmarke gefüllt worden ist, wird ein Verschlußkäppchen 6′ aufgesteckt, da die Messung der BSG unter Luftabschluß erfolgen muß. Die Entnahmespritze 1 wird anschließend mit ihrem Senkungsrohr 9 an einem Gestell 28 befestigt, das zu diesem Zweck mit einer Klemme 29 versehen ist. Das Gestell 28 ist dabei mit einer graduierten Skala 30 versehen, anhand der die BSG gemessen werden kann.

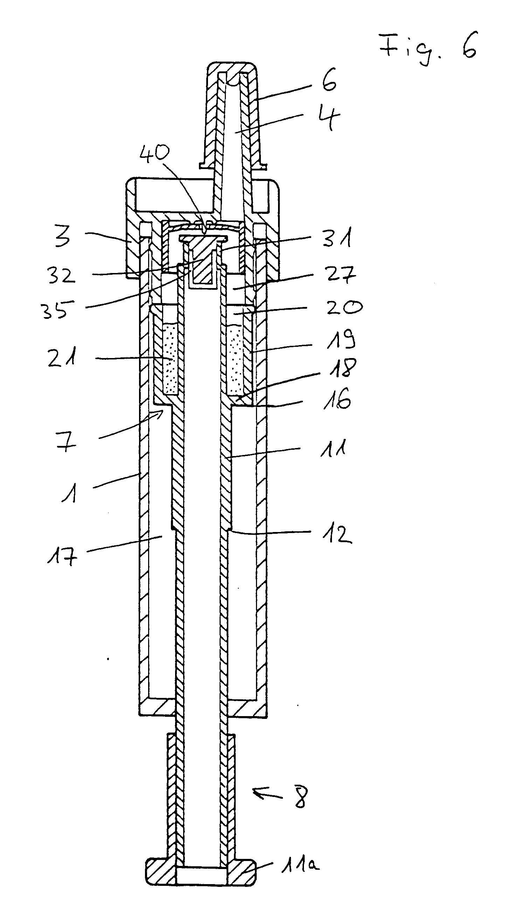

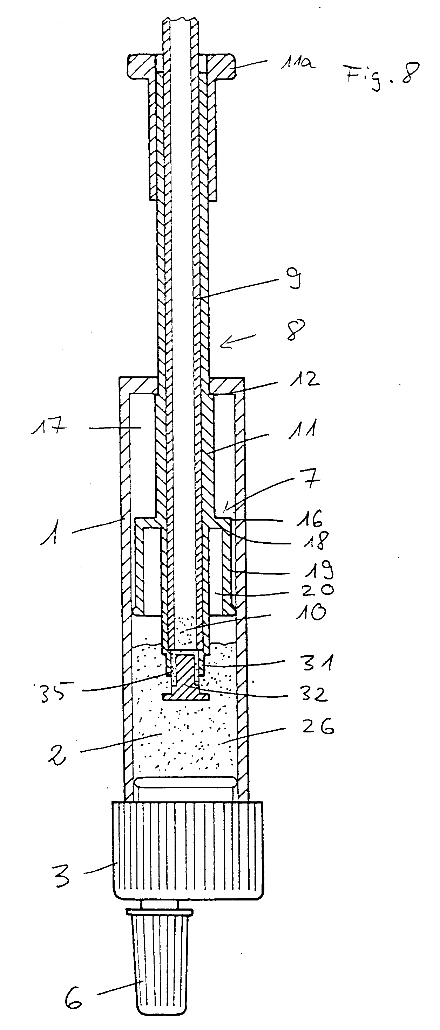

Die Ausführungsform in den Fig. 6 bis 11 entspricht im wesentlichen der in den Fig. 1 bis 5 dargestellten ersten Ausführungsform. Der Hauptunterschied zur ersten Ausführungsform liegt jedoch darin, daß das Senkungsrohr 9 in die Hülse 11 hineinsteckbar und wieder herausziehbar ist, wobei in Fig. 6 die Entnahmespritze 1 ohne Senkungsrohr 9 und in Fig. 8 die Entnahmespritze 1 mit Senkungsrohr dargestellt ist. Bei herausgezogenem Senkungsrohr 9 bildet somit die Hülse 11 alleinig die Spritzenkolbenstange 8 (sowie den Spritzenkolben 7).

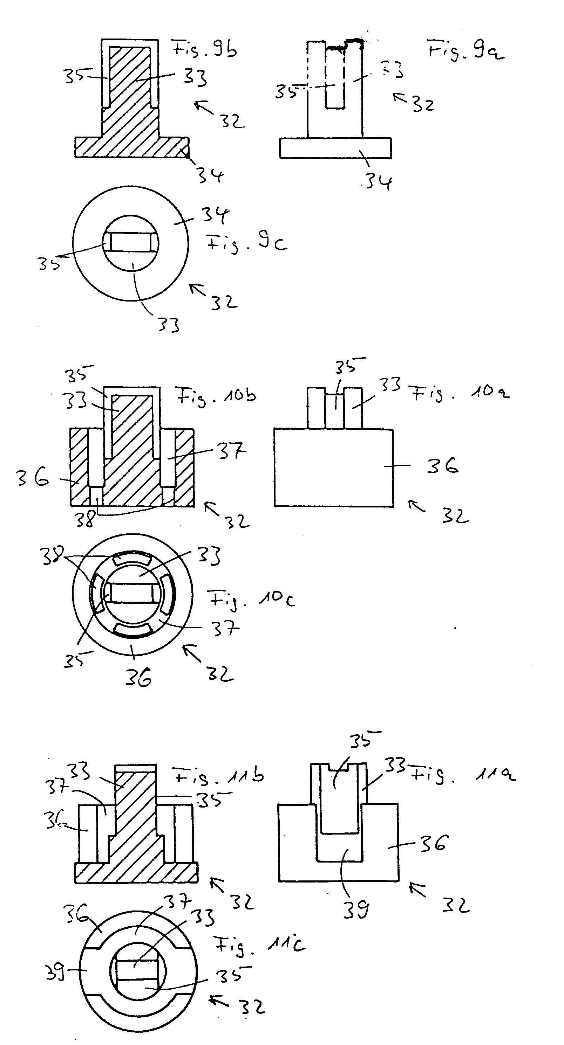

Ein weiterer Hauptunterschied zur ersten Ausführungsform besteht darin, daß statt der Ringscheibe 13 bei dieser zweiten Ausführungsform ein Hohlzylinder 31 am vorderen Ende der Hülse 11 angeformt ist, wobei auch dieser Hohlzylinder 31 eine mittige Öffnung 14 besitzt. In diese Öffnung 14 ist ein Verschlußstopfen 32 eingesetzt. Verschiedene Ausführungsformen dieses Verschlußstopfens 32 sind in den Fig. 9 bis 11 dargestellt, wobei der Verschlußstopfen in Fig. 9 bei der Entnahmespritze 1 in den Fig. 6 bis 8 verwendet ist. Somit soll zunächst dieser beschrieben werden.

Der Verschlußstopfen in Fig. 9 ist im wesentlichen entsprechend der Öffnung 14 zylindrisch ausgebildet, wobei das obere Ende des Schaftes 33 durch einen Anschlagring 34 abgeschlossen ist. Weiterhin weist der Schaft 33 des Verschlußstopfens 32 einen Durchströmkanal 35 in Form von zur Schaftachse parallele Nuten auf, die jedoch nicht bis zum Anschlagring 34 reichen.

Der Verschlußstopfen 32 in Fig. 10 ist ähnlich aufgebaut. Er besitzt zunächst ebenfalls einen Schaft 33 mit einem Durchströmkanal 35 in Form von parallel zur Schaftachse verlaufenden Nuten. Statt eines Anschlagringes 34 weist der Verschlußstopfen 32 jedoch hier eine Kappe 36 auf, welche mit dem Schaft 33 eine Ringausnehmung 37 definiert, welche im Bodenbereich Durchbrechungsschlitze 38 aufweist.

Ähnlich ist auch der Verschlußstopfen 32 in Fig. 11 ausgebildet. Hier weist der Schaft 33 ebenfalls einen Durchströmkanal 35 auf, der jedoch breiter ausgebildet ist als die Durchströmkanäle in den Fig. 9 und 10. Vor allem aber weist diese Ausführungsform ebenfalls eine Kappe 36 auf, die ebenfalls mit dem Schaft 33 eine Ringausnehmung 37 definiert. An zwei gegenüberliegenden Seiten weist die Kappe 36 jedoch Ausnehmungen 39 auf.

Der Verschlußstopfen aus Fig. 9 ist bei der in den Fig. 6 bis 8 dargestellten Entnahmespritze 1 im Ausgangszustand vollständig in die Öffnung 14 des Hohlzylinders 31 hineingesteckt. Der Anschlagring 34 kommt dabei am vorderen Ende des Hohlzylinders 31 zur Anlage. Da die Durchströmkanäle 35 nicht ganz bis hin zum Anschlagring 34 reichen, liegt der Schaft 33 im Bereich des Anschlagringes 34 um den ganzen Umfang herum an der Innenwand der Öffnung 14 des Hohlzylinders 31 an und verschließt diese somit dichtend. In diese Stellung wird der Verschlußstopfen 32 durch einen Anschlagstift 40 gedrückt, der am Anschlußstück 3 die Ventilöffnung 23 durchragend angeformt ist.

In entsprechender Weise ist der Verschlußstopfen 32 aus Fig. 10 auf den Hohlzylinder 31 aufgesteckt, wobei der Hohlzylinder 31 in der Ringausnehmung 37 zu liegen kommt. Um beim Aufschieben auf den Hohlzylinder 31 einen Luftstau zu vermeiden, sind die Durchbrechungsschlitze 38 vorgesehen.

Entsprechend ist der Verschlußstopfen 32 aus Fig. 11 auf den Hohlzylinder 31 aufgesteckt, wobei hier die Ausnehmungen 39 einen Luftstau vermeiden.

Bei sämtlichen Ausführungsformen des Verschlußstopfens 32 dichten diese im voll aufgeschobenen Zustand die Öffnung 14 im Hohlzylinder 31 ab.

Die Arbeits- und Funktionsweise dieser zweiten Ausführungsform einer Vorrichtung zum Messen der BSG wird ebenfalls nachfolgend beschrieben:

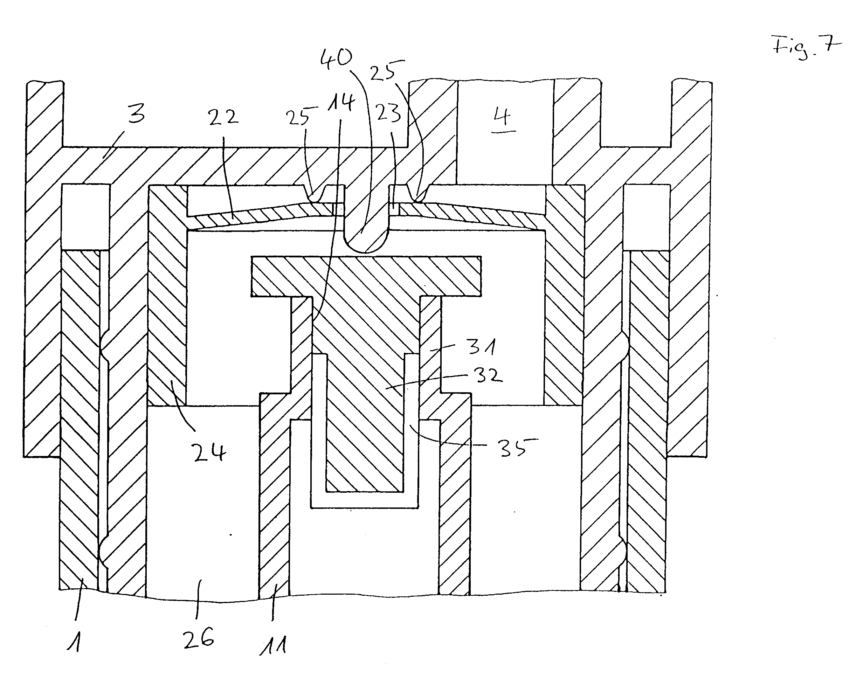

Der Ursprungszustand der Entnahmespritze 1 ist in Fig. 6 (sowie in Fig. 7) dargestellt. Der Verschlußstopfen 32 ist voll in den Hohlzylinder 31 eingeschoben und verschließt die Öffnung 14. Das Senkungsrohr 9 ist dabei nicht in die Hülse 11 eingeschoben.

Nach Entfernen des Verschlußkäppchens 6 von der Aufziehöffnung 4 und nach Aufstecken einer Kanüle kann dem Patienten eine Blutprobe entnommen werden. Dabei wird die Hülse 11 nach hinten gezogen, so daß die Blutprobe 2 in die Probenkammer 26 angesaugt wird, indem die Membrane 22 vom Ventilsitz 25 abhebt und somit das Rückschlagventil geöffnet wird. Die Hülse 11 gelangt dabei mit ihrem Anschlag 12 bis an das rückwärtige Ende der Entnahmespritze 1, so daß in dieser Position die vorgegebene Menge der Blutprobe 2 aufgezogen ist. Während dieses gesamten Vorganges verschließt der Verschlußstopfen 32 die Öffnung 14 im Hohlzylinder 31.

Nach Durchmischen der Blutprobe 2 mit dem Blutgerinnungshemmer 21 kann dann das Umfüllen in das Senkungsrohr 9 erfolgen. Dieses wird zunächst in die Hülse 11 bis hin zum Hohlzylinder 31 geschoben, welcher als Anschlag dient. Dabei wird der Verschlußstopfen 32 durch das untere Ende 10 des Senkungsrohres 9 nach außen gedrückt derart, daß der Durchströmkanal 35 teilweise außerhalb des Hohlzylinders 31 zu liegen kommt und somit den Weg zwischen der Probenkammer 26 und dem Innern des Senkungsrohres 9 freigibt. Dieser Zustand ist in Fig. 8 dargestellt. Durch Verschieben der Hülse 11 (zusammen mit dem nunmehr fest darin angeordneten Senkungsrohr 9) entgegen der Aufziehrichtung wird die ungerinnbar gemachte Probe 2 in das Senkungsrohr 9 umgefüllt, indem die Blutprobe 2 durch die Durchströmkanäle 35 des Verschlußstopfens 32 aus der Probenkammer 26 ins Innere des Senkungsrohres 9 fließt. Sobald die Hülse 11 wieder ihre Ausgangsposition erreicht, bei der die vorgeschriebene Menge der Blutprobe 2 in das Senkungsrohr 9 umgefüllt worden ist, kommt der Verschlußstopfen 32 am Anschlagstift 40 zur Anlage und drückt den Verschlußstopfen 32 wieder nach innen, so daß er die Öffnung 14 des Hohlzylinders 31 wieder verschließt. Somit ist das untere Ende des Senkungsrohres 9 dichtend verschlossen, so daß die Messung der Blutkörperchensenkungsgeschwindigkeit durchgeführt werden kann, wie dies in entsprechender Weise in Fig. 3 dargestellt ist.

Die Arbeits- und Funktionsweise wurde anhand des Verschlußstopfens erläutert, wie er in Fig. 9 dargestellt ist. Die Funktionsweise unter Verwendung der Verschlußstopfen 32 in den Fig. 10 und 11 erfolgt dabei in analoger Weise.

| **Bezugszeichenliste** | | | |
|---|---|---|---|
| 1 | Entnahmespritze | 31 | Hohlzylinder |
| 2 | Blutprobe | 32 | Verschlußstopfen |
| 3 | Anschlußstück | 33 | Schaft |
| 4 | Aufziehöffnung | 34 | Anschlagring |
| 5 | Kanüle | 35 | Durchströmkanal |
| 6 | Verschlußkäppchen | 36 | Kappe |
| 6′ | Verschlußkäppchen | 37 | Ringausnehmung |
| 7 | Spritzenkolben | 38 | Durchbrechungsschlitz |
| 8 | Spritzenkolbenstange | 39 | Ausnehmung |
| 9 | Senkungsrohr | 40 | Anschlagstift |
| 10 | unteres Ende | | |
| 10′ | oberes Ende | | |
| 11 | Hülse | | |
| 11a | Handhabe | | |
| 12 | Anschlag | | |
| 13 | Ringscheibe | | |
| 14 | Öffnung | | |
| 15 | Verschlußstift | | |
| 16 | Erweiterung | | |
| 17 | Ringraum | | |
| 18 | Ringscheibe | | |
| 19 | Zylindermantel | | |
| 20 | Tasche | | |
| 21 | Blutgerinnungshemmer | | |
| 22 | Membrane | | |
| 23 | Ventilöffnung | | |
| 24 | Haltering | | |
| 25 | Ventilsitz | | |
| 26 | Probenkammer | | |
| 27 | Luftpolster | | |
| 28 | Gestell | | |
| 29 | Klemme | | |
| 30 | Skala | | |

## Patentansprüche

1. Vorrichtung zum Messen der Blutkörperchensenkungsgeschwindigkeit (BSG) einer ungerinnbar gemachten Blutprobe (2) mittels eines Senkungsrohres (9) unter Verwendung einer Entnahmespritze (1), wobei das Senkungsrohr (9) zugleich auch die den Spritzenkolben (7) tragende Spritzenkolbenstange (8) der Entnahmespritze (1) bildet und wobei zum Umfüllen der aufgezogenen Blutprobe (2) diese beim Verschieben des Spritzenkolbens (7) in die umgekehrte Aufziehrichtung von der Probenkammer (26) der Entnahmespritze (1) in das Senkungsrohr (9) gedrückt wird, wobei die Vorrichtung in einem Gestell (28) mit daran angebrachter Ableseskala (30) angeordnet ist,
**dadurch gekennzeichnet,**
daß das Senkungsrohr (9) im Nichtablesebereich des Gestells (28) von einer die eigentliche Spritzenkolbenstange (8) mit dem Spritzenkolben (7) bildenden Hülse (11) umgeben ist,
daß die Hülse (11) an ihrem unteren Ende durch einen eine mittige Öffnung (14) aufweisenden, angeformten Ansatz abgeschlossen ist, bis zu dem das Senkungsrohr (9) in die Hülse (11) hineinsteckbar ist, und
daß der Öffnung (14) ein diese im hineingeschobenen Zustand des Spritzenkolbens (7) verschließendes Verschlußelement zugeordnet ist.

2. Vorrichtung nach Anspruch 1 , dadurch gekennzeichnet, daß der Ansatz durch eine Ringscheibe (13) gebildet ist, daß das Senkungsrohr (9) dauerhaft von der Hülse (11) aufgenommen ist und daß im Boden der Entnahmespritze (1) ein ins Innere der Probenkammer (26) ragender, das Verschlußelement bildender Verschlußstift (15) angeordnet ist, der in der unteren Position des Senkungsrohres (9) die Öffnung (14) dichtend verschließt.

3. Vorrichtung nach Anspruch 1, dadurch gekennzeichnet, daß der Ansatz durch einen Hohlzylinder (31) gebildet ist, daß das Senkungsrohr (9) bis zum Hohlzylinder (31) in die Hülse (11) hineinsteckbar und wieder herausziehbar ist und daß in der Öffnung (14) des Hohlzylinders (31) ein durch Hineinstecken des Senkungsrohres (9) in die Hülse (11) sowie durch Anlage am Boden der Entnahmespritze (1) entsprechend verschiebbarer, das Verschlußelement bildender Verschlußstopfen (32) angeordnet ist, der in der durch das Senkungsrohr (9) hinausgedrückten Verschiebestellung einen Durchströmkanal (35) zwischen dem Innern des Senkungsrohres (9) und der Probenkammer (26) öffnet und ansonsten verschließt.

4. Vorrichtung nach Anspruch 3, dadurch gekennzeichnet, daß der Durchströmkanal (35) im Verschlußstopfen (32) ausgebildet ist.

5. Vorrichtung nach Anspruch 3 oder 4, dadurch gekennzeichnet, daß im Boden der Entnahmespritze (1) ein Anschlagstift (40) für den Verschlußstopfen (32) angeordnet ist.

6. Vorrichtung nach einem der Ansprüche 1 bis 5, dadurch gekennzeichnet, daß die Hülse (11) im Innern der Entnahmespritze (1) einen Anschlag (12) aufweist.

7. Vorrichtung nach einem der Ansprüche 1 bis 6, dadurch gekennzeichnet, daß die Hülse (11) außerhalb der Entnahmespritze (1) eine Handhabe (11a) aufweist.

8. Vorrichtung nach einem der Ansprüche 1 bis 7, dadurch gekennzeichnet, daß der Spritzenkolben (7) durch eine Erweiterung (16) der Hülse (11) gebildet ist.

9. Vorrichtung nach Anspruch 8, dadurch gekennzeichnet, daß die Erweiterung (16) der Hülse (11) eine zur Probenkammer (26) hin offene zylinderringförmige Tasche (20) zur Aufnahme eines Blutgerinnungshemmers (21) aufweist.

10. Vorrichtung nach einem der Ansprüche 1 bis 9, dadurch gekennzeichnet, daß zum Verschließen der Aufziehöffnung (4) der Entnahmespritze (1) ein Rückschlagventil vorgesehen ist.

11. Vorrichtung nach Anspruch 10, dadurch gekennzeichnet, daß das Rückschlagventil aus einer elastisch verformbaren Membrane (22) mit einer Ventilöffnung (23) sowie einem im Boden der Entnahmespritze (1) angeformten, dazu korrespondierenden Ventilsitz (25) besteht.

## Claims

1. Device for measuring the blood corpuscle sedimentation rate (BSR) of a blood sample (2), which has been rendered non-coagulable, by means of a sedimentation tube (9), using a collecting syringe (1), where the sedimentation tube (9) at the same time also forms the syringe piston rod (8), carrying the syringe piston (7), of the collecting syringe (1) and where, for transferring the drawn-up blood sample (2), the latter is pushed, on displacing the syringe piston (7) in the reversed drawing-up direction, from the sample chamber (26) of the collecting syringe (1) into the sedimentation tube (9), the device being arranged on a stand (28) to which is affixed a reading scale (30), characterised in that the sedimentation tube (9), in the non-reading region of the stand (28), is surrounded by a casing (11) which forms the actual syringe piston rod (8) together with the syringe piston (7), in that the casing (11), at its bottom end, is closed off by a moulded-on shoulder having a central opening (14), up to which shoulder the sedimentation tube (9) can be inserted into the casing (11), and in that an occluding element is allocated to the opening (14), which element occludes the opening in the pushed-in condition of the syringe piston (7).

2. Device according to Claim 1, characterised in that the shoulder is formed by a ring wheel (13), in that the sedimentation tube (9) is permanently taken up by the casing (11) and in that an occluding peg (15), which projects into the interior of the sample chamber (26) and which forms the occluding element, is arranged in the floor of the collecting syringe (1), which peg occludes the opening (14) in a sealing manner in the bottom position of the sedimentation tube (9).

3. Device according to Claim 1, characterised in that the shoulder is formed by a hollow cylinder (31), in that the sedimentation tube (9) can be inserted into the casing (11) up to the hollow cylinder (31), and can be pulled out again, and in that an occluding stopper (32), which forms the occluding element and which is correspondingly displaceable by insertion of the sedimentation tube (9) into the casing (11) and by abuttal on the floor of the collecting syringe (1), is arranged in the opening (14) of the hollow cylinder (31), which stopper, when it is pushed out into its displacement position by the sedimentation tube (9), opens a flow-through channel (35) between the interior of the sedimentation tube (9) and the sample chamber (26), and otherwise occludes it.

4. Device according to Claim 3, characterised in that the flow-through channel (35) is formed in the occluding stopper (32).

5. Device according to Claim 3 or 4, characterised in that a limit-stop peg (40) for the occluding stopper (32) is arranged in the floor of the collecting syringe (1).

6. Device according to one of Claims 1 to 5, characterised in that the casing (11) possesses a limit stop (12) in the interior of the collecting syringe (1).

7. Device according to one of Claims 1 to 6, characterised in that the casing (11) possesses a handle (11a) outside the collecting syringe (1).

8. Device according to one of Claims 1 to 7, characterised in that the syringe piston (7) is formed by a widening (16) of the casing (11).

9. Device according to Claim 8, characterised in that the widening (16) of the casing (11) possesses a cylindrically annular pocket (20), which is open towards the sample chamber (26), for receiving a blood coagulation inhibitor (21).

10. Device according to one of Claims 1 to 9, characterised in that a nonreturn valve is provided for occluding the drawing-up opening (4) of the collecting syringe (1).

11. Device according to Claim 10, characterised in that the nonreturn valve consists of an elastically deformable membrane (22), having a valve opening (23), and of a valve seat (25) corresponding thereto, which seat is moulded on in the floor of the collecting syringe (1).

## Revendications

1. Dispositif de mesure de la vitesse de sédimentation globulaire (VSG) d'un prélèvement sanguin rendu incoagulable (2), au moyen d'un tube à sédimentation (9), en utilisant une seringue de prélèvement (1), le tube à sédimentation (9) formant simultanément la tige de piston de seringue (8) de la seringue de prélèvement (1), ladite tige portant le piston de seringue (7), le prélèvement sanguin aspire (2) étant refoulé, pour être transvasé, de la chambre d'échantillon (26) de la seringue de prélèvement (1) dans le tube à sédimentation (9) par un coulisse-ment du piston de seringue (7) dans le sens d'aspiration inverse, le dispositif étant disposé dans un bâti (28) sur lequel est placée une échelle de mesure (30), caractérisé en ce que, dans la plage hors lecture du bâti (28), le tube à sédimentation (9) est entouré par une douille (11) formant, avec le piston de seringue (7), la tige de piston de seringue proprement dite (8), en ce que, à son extrémité inférieure, la douille (11) est fermée par un embout moulé qui comporte une ouverture centrale (14) et jusqu'auquel le tube à sédimentation (9) peut être enfoncé dans la douille (11), et en ce qu'à l'ouverture (14) est associé un élément d'obturation qui la ferme lorsque le piston de seringue (7) est enfoncé.

2. Dispositif selon la revendication 1, caractérisé en ce que l'embout est formé par une rondelle annulaire (13), en ce que le tube à sédimentation (9) est logé à demeure dans la douille (11) et en ce que, au fond de la seringue de prélèvement (1), est disposée une broche d'obturation (15) qui pénètre à l'intérieur de la chambre d'échantillon (26) et forme l'élément d'obturation et qui, lorsque le tube à sédimentation (9) est en position basse, ferme l'ouverture (14) de manière étanche.

3. Dispositif selon la revendication 1, caractérisé en ce que l'embout est formé par un cylindre creux (31), en ce que le tube à sédimentation (9) peut être introduit dans la douille (11) jusqu'au cylindre creux (31) et peut en être retiré, et en ce que, dans l'ouverture (14) du cylindre creux (31), est disposé un bouchon d'obturation (32) qui forme l'élément d'obturation et peut coulisser de manière correspondante lors de l'enfoncement du tube à sédimentation (9) dans la douille (11) ainsi que lors de sa venue en butée contre le fond de la seringue de prélèvement (1), et qui, dans la position de coulissement où il est poussé vers l'extérieur par le tube à sédimentation (9), ouvre un canal d'écoulement (35) entre l'intérieur du tube à sédimentation (9) et la chambre d'échantillon (26), et sinon le ferme.

4. Dispositif selon la revendication 3, caractérisé en ce que le canal d'écoulement (35) est formé dans le bouchon d'obturation (32).

5. Dispositif selon la revendication 3 ou 4, caractérisé en ce que, dans le fond de la seringue de prélèvement (1), est disposée une broche de butée (40) pour le bouchon d'obturation (32).

6. Dispositif selon l'une des revendications 1 à 5, caractérisé en ce que la douille (11) comporte une butée (12) à l'intérieur de la seringue de prélèvement (1).

7. Dispositif selon l'une des revendications 1 à 6, caractérisé en ce que la douille (11) comporte, à l'extérieur de la seringue de prélèvement (1), une poignée (11a).

8. Dispositif selon l'une des revendications 1 à 7, caractérisé en ce que le piston de seringue (7) est formé par un élargissement (16) de la douille (11).

9. Dispositif selon la revendication 8, caractérisé en ce que l'élargissement (16) de la douille (11) comporte une poche en forme d'anneau cylindrique (20) ouverte en direction de la chambre d'échantillon (26) et destinée à recevoir un anticoagulant (21).

10. Dispositif selon l'une des revendications 1 à 9, caractérisé en ce que, pour fermer l'ouverture d'aspiration (4) de la seringue de prélèvement (1), il est prévu une soupape anti-retour.

11. Dispositif selon la revendication 10, caractérisé en ce que la soupape anti-retour se compose d'une membrane déformable élastiquement (22), pourvue d'une ouverture de soupape (23), ainsi que d'un siège de soupape correspondant (25) moulé dans le fond de la seringue de prélèvement (1).
